# EUROPEAN PATENT SPECIFICATION

(11) **EP 3 357 464 B1**
(45) Date of publication and mention of the grant of the patent: **10.07.2019**
(21) Application number: 16851111.1
(22) Date of filing: 12.09.2016
(51) Int. Cl.: A61F 13/49, A61F 13/494, A61F 13/534

(54) **UNDERPANTS-TYPE DIAPER**
UNTERHOSENARTIGE WINDEL
COUCHE DE TYPE SOUS-VÊTEMENT

(30) Priority: 02.10.2015 JP 2015197241
(43) Date of publication of application: 08.08.2018
(73) Proprietor: Unicharm Corporation, Ehime 799-0111 (JP)
(72) Inventor: MUKAI, Hirotomo, Kanonji-shi Kagawa 769-1602 (JP); NINOMIYA, Akihide, Kanonji-shi Kagawa 769-1602 (JP); SHIOMI, Akihisa, Kanonji-shi Kagawa 769-1602 (JP)
(74) Representative: Staeger & Sperling Partnerschaftsgesellschaft mbB
(86) International application number: PCT/JP2016/076772
(87) International publication number: WO 2017/056937

(56) References cited:
- EP-A1- 1 197 195
- EP-A1- 2 221 031
- EP-A1- 2 556 809
- EP-A1- 2 561 847
- JP-A- 2005 312 557
- JP-A- 2007 029 507
- JP-A- 2008 229 007
- JP-A- 2011 217 899
- JP-A- 2011 240 054
- US-A1- 2013 041 340

## Description

### [TECHNICAL FIELD]

The present invention relates to pants-type diapers.

### [BACKGROUND ART]

A pants-type diaper known in the art includes an elastic member (elastic member for fit gathers) which is stretchable in the width direction of a front waistline member. The elastic member is cut in the center region (a region where an absorber is disposed) in the width direction of the front waistline member to partially decrease stretchability of the elastic member in the width direction to maintain the absorber to a flat state as much as possible.

Thus, an effective absorbing width of the absorber can be increased (see Patent Literature 1).

### [CITATION LIST]

### [PATENT LITERATURE]

Patent Literature 1: JP 2014-221122 A (Paragraph 0031)
Patent Literature 2: JP2002-119537 (Claim 1)

Further prior art in this technical field is disclosed in documents EP 2 561 847 A1 and EP 2 556 809 A1.

### [SUMMARY OF INVENTION]

However, simply cutting the elastic member, which is provided at least in the front or rear waistline member, in the center region of the front waistline member in the width direction only leads to dispose elastic members at equal intervals over the entire width between the absorber and a side edge of the front or rear frontline member. Thus, it is difficult to adjust stretchability of the elastic member in the width direction.

Patent Literature 2 discloses that at least the front or rear waistline member is divided into multiple regions in the width direction. Extension stress in a first stretch region determined as a region between the side edge of a liquid absorbing core and the side edge of the front or rear waistline member is set larger than extension stress in a second stretch region which is determined to be a region between both side edges of the liquid absorbing core. This technique, however, is directed to establish the extension stress, although the extension stress is relatively small, in the region overlapping the liquid absorbing core. The technique therefore does not aim to positively decrease the stretchability of the absorber due to the elastic member.

The present invention is provided to solve the above problem and, it is an object of the present invention to provide a pants-type diaper that can achieve a wider effective absorbing range of the absorber, adjust stretchability of the elastic members in the width direction, and improve the wearing feeling of the absorber.

The present invention is defined by the features of claim 1 and refers to a pants-type diaper, including: a width direction and a longitudinal direction which are orthogonal to each other; a front waistline member; a rear waistline member; an absorber body extending from the front waistline member to the rear waistline member; an absorber disposed in the absorber body; and a plurality of elastic members disposed at predetermined intervals in the longitudinal direction in the front waistline member and the rear waistline member, wherein at least one of the front waistline member and the rear waistline member includes a first region including both side edges along the longitudinal direction of the region where the absorber is disposed and outside in the width direction, and a second region provided outside the first region in the width direction, and extension stress of the first region is smaller than extension stress of the second region.

### [BRIEF DESCRIPTION OF DRAWINGS]

Fig. 1 illustrates an external appearance of an example pants-type diaper according to a first embodiment of the present invention.
Fig. 2 is a plan view (on a skin-abutting surface side) of an example pants-type diaper according to the first embodiment of the present invention when cut and developed at a bonded portion.
Fig. 3 is a cross-sectional view cut along line X-X of Fig. 2.

### [DESCRIPTION OF EMBODIMENTS]

### (First Embodiment)

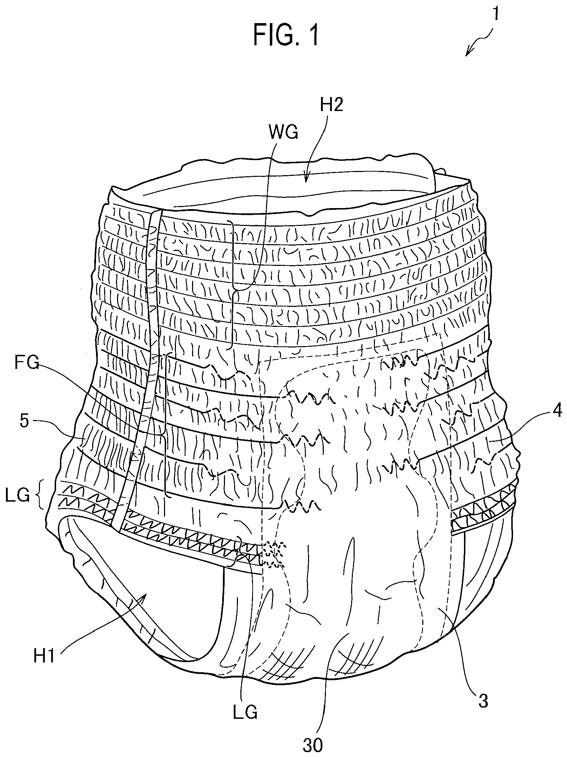

A pants-type diaper 1 according to a first embodiment of the present invention is described by referring to FIGS. 1 to 3.

In the drawings, the same or similar parts are indicated by the same or similar reference symbols. The drawings are illustrated schematically, and dimensional ratio and other variables differ from those of actual measurements.

As illustrated in FIGS. 1 to 3, a pants-type diaper 1 according to the present embodiment has a width direction W and a longitudinal direction orthogonal to each other, and includes a front waistline member (front body) 4, an absorber body (inner body) 3, a rear waistline member (rear body) 5, an absorber (absorber body) 30, and a plurality of elastic members WG, FG, and LG.

In the pants-type diaper 1 according to the present embodiment includes both side units 50E of the front and rear waistline members 4 and 5 are bonded to form a waist aperture H2 and leg apertures H1.

The absorber body 3 extends to the front and rear waistline members 4 and 5. For example, the absorber body 3 may be disposed overlapping the front and rear waistline members 4 and 5, or may overlap at least one of the front and rear waistline members 4 and 5. The absorber 30 is disposed on the absorber body 30.

The elastic members WG, FG, and LG are each disposed at predetermined intervals in the longitudinal direction L in the front and rear waistline members 4 and 5. The elastic members WG are the elastic members (waistline elastic members) disposed on the waist aperture H2 side to form waist gathers. The elastic members FG are the elastic members that form fit gathers. The elastic members LG are the elastic members (leg-opening elastic members) disposed at the leg-aperture H1 side to form leg gathers.

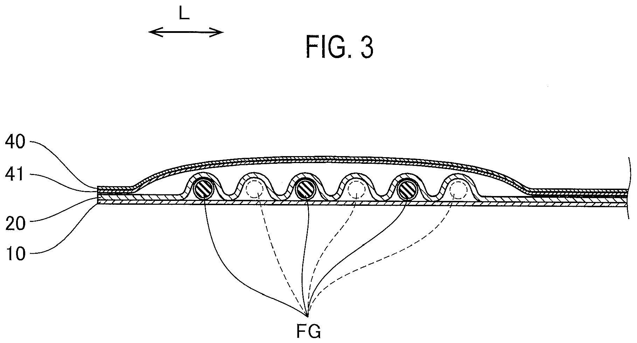

As illustrated in FIG. 3, the elastic members WG, FG, and LG are stacked with adhesives between a topsheet 20 and a backsheet 10 (a pair of sheet members) in the front and rear waistline members 4 and 5.

In an absorber body 3, the absorber 30 is stacked with an adhesive between topsheet 40 and a backsheet 41 (a pair of sheet members).

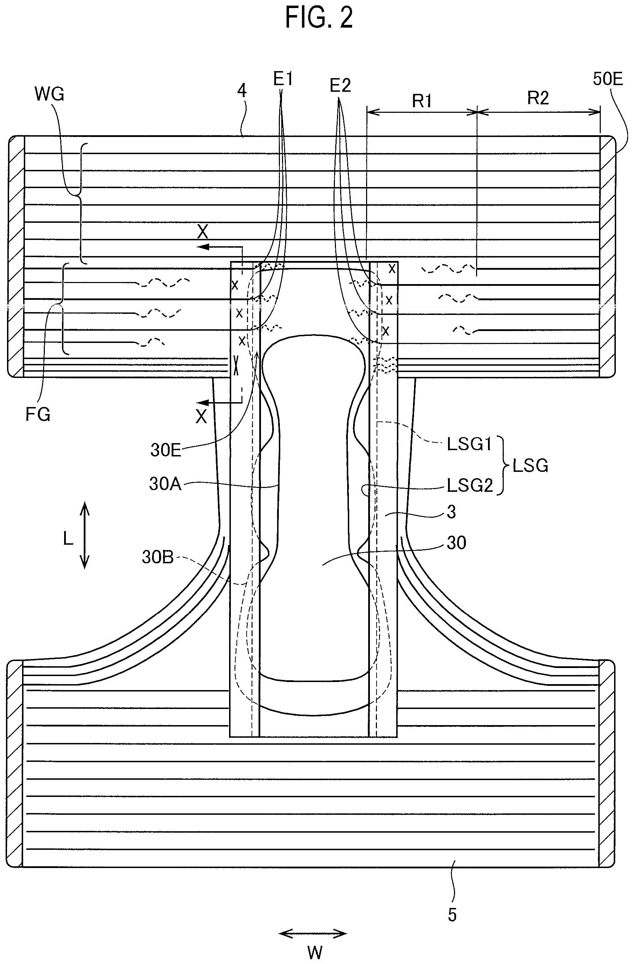

As illustrated in FIG. 2, at least one of the front and rear waistline portions 4 and 5 includes a first region R1 and a second region R2. In the following description of the pants-type diaper 1 according to the present embodiment, the first and second regions R1 and R2 are formed only in the front waistline member 4, but the embodiment of the present invention is not limited to this case.

The first region R1 is a region including both side edges 30E along the longitudinal direction L of the region where the absorber 30 is disposed and outside in the width direction W. The second region R2 is a region formed outside of the first region R1 in the width direction W. In the present embodiment, it is assumed that both side edges 30E include regions each about 10 mm inside from the outer edges of the absorber 30 in the width direction W.

In the pants-type diaper 1 according to the present embodiment, the first region R1 has extension stress set smaller than extension stress of the second region R2. For example, the extension stress of the first region R1 may be 0.05 to 1.5 N/25 mm and the elastic stress of the second region R2 may be 0.3 to 3.0 N/25 mm.

In other words, a stronger extension stress is necessary to achieve 60% extension in the first region R1 than in the second region R2. In use, hands are inserted into the inside of the side seal portions on both sides for left and right expansion. At this time, the first region R1 first reaches 60% extension size when the second region R2 is on the way to reach 60% extension.

In particular, the first and second regions R1 and R2 have been extended equally when worn by a person who has a relatively narrow waist size. In contrast, the first region R1 is extended further when worn, so that both side edges 30E of the absorber 30 can also be extended further outward. Thus, the absorber can be fit widely with less tucking, while the elastic members FG of both side edges 30E of the absorber 30 are further extended to enable pressing more strongly. This can minimize a gap against the body that is generated when the body moves, whereby the leakage can be prevented.

Measuring the extension stress of the first and second regions R1 and R2 is briefly described below.

In a first step, the pants-type diaper 1 is extended in the longitudinal direction L and the width direction W to straighten wrinkles. In this state, line A is drawn in front or rear waistline member 4 or 5 in the longitudinal direction L along the outer side edge of the absorber 30 in the width direction W between the left or right side seal portion and the outer side edge 30E on the left or right side of the side seal portion in the width direction W of the absorber 30. Line B is drawn in the longitudinal direction L inside the side seal portion in the width direction W. Line C is drawn in the longitudinal direction L so as to form two regions including a region (1) including the first region R1 on the outer side edge 30E side in the width direction W of the absorber 30 and a region (2) including the second region R2 on the side seal portion side.

In a second step, a chuck gripping portion with a tensile tester is marked. Lines D/E are drawn in the longitudinal direction L with a 20 mm width inside and outside of the line C in the width direction W. Line F is drawn in parallel with the line B at 20 mm inside the line B in the width direction.

In a third step, the chuck gripping positions are determined. In the region (1) including the first region R1, one chuck gripping position is determined at a position located inside the absorber 30 in the width direction W with the line A regarded as an inner reference line for the chuck. The other chuck gripping position is determined between the lines D and C with the line D being the inner reference line for the chuck. In the region (2) including the second region R2, one chuck gripping position is determined between the lines E and C with the line E regarded as an inner reference line for the chuck. The other chuck gripping position is determined at a position located outside the line F in the width direction W with the line F being the inner reference line for the chuck.

In a fourth step, a predetermined length of a region of the pants-type diaper 1 where the elastic members FG are disposed is taken out in the longitudinal direction L such that the reference line and a sufficient amount of margin to be gripped by the chuck are included in the width direction W. The taken-out portion is then cut along the line C and divided into the first and second regions R1 and R.

In a fifth step, the tensile tester is set at each chuck gripping position to measure the tensile strength. A distance between the chucks is set smaller than a distance in the width direction W of the region to be measured than a distance when the region to be measured is best shrunken. A two-cycle test is carried out until 90% of the distance relative to the maximum length between the lines A and D and the lines E and F is reached. Then, the strength of the second test at 60% extension relative to the maximum length is read for comparison.

The reason why the strength at 60% extension is read is that this measurement can assume a situation where approximately middle-sized persons among the adaptive range of target persons wear the diaper. If the set stretch magnification is low and the shrinking length relative to the maximum length increases, the reading and comparison is not limited to the extension stress at 60% extension, and may be carried in the range of 30% to 60% extension, or at more than 60% extension.

Meanwhile, the above-described method can also be used to measure the first and second regions R1 and R2 of the other side (left or right side). For example, the Autograph tensile tester (Model: AG-X10plus) manufactured by SHIMADZU CORPORATION can be used.

Specifically, as illustrated in FIG. 2, in the pants-type diaper according to the present embodiment, the elastic members FG may extend in the width direction W over the first and second regions R1 and R2, or may extend in the width direction W only in the second region R2.

In FIG. 2, solid lines indicate portions of the elastic members FG that are stretchable (stretchable region), as the elastic members FG are bonded to the backsheet 10 (and the topsheet 20). Broken lines indicate portions of the elastic members FG that are non-stretchable (non-stretchable region), as the elastic members FG are not bonded to the backsheet 10 (or the topsheet 20).

As used herein, the non-stretchable region of the elastic members FG means that the elastic members FG are not fixed in a stretched state. It is assumed that the stretching ratio of up to about 10% is regarded as not being stretched. The stretching ratio is determined by first stretching and fixing the portion of the pants-type diaper 1 where the elastic members FG are present in the longitudinal direction L and the width direction W until no wrinkles exist. Then, the entire member disposed overlapping the elastic members FG is cut out for a fixed length of A mm in the width direction W of the pants-type diaper 1. Only the elastic members FG included in the cut portion is soaked in toluene, for example. The cut portion is then taken out and dried and, after drying, a length B mm is measured. Thus, the stretching ratio is (A/B × 100 - 100) %. If a negative value is obtained, the stretching ratio is regarded as zero. That is, in such a non-stretching region, the elastic member FG is not bonded to the topsheet 20 and the backsheet 10 of the front waistline member 4 in the stretched state.

In this structure, the regions (the first and second regions R1 and R2) in which the elastic members FG are disposed in the front waistline member 4 are provided outside the absorber 30 including the both side edges 30E in the width direction W.

Therefore, the elasticity achieved by the elastic members FG decrease in the region where the absorber 30 is disposed, whereby the formation of wrinkles in the absorber 30 can be prevented and a wider effective absorbing range of the absorber 30 can be achieved.

In addition, this structure decreases the extension stress of the first region R1, while maintaining the extension stress in the second region R2. Thus, the stretchability can be adjusted in the width direction W. As a result, the pants-type diaper 1 can be worn by first expanding the first region R1 having a small extension stress of the pants-type diaper 1. This prevents formation of wrinkles in an extended area including the absorber 30, allowing the pants-type diaper 1 to be worn with the absorber 30 being widely expanded. Thus, the wearing feeling of the absorber 30 can be improved.

In the pants-type diaper 1 according to the present invention, end portions E1 and E2 inside the elastic members FG in the width direction W in the first region R1 is disposed shifted from each other in the longitudinal direction L between one outer side (for example, the left side of the both side edge 30E) including the both side edge 30E in the width direction W and the other outer side (for example, the right side of the both side edge 30E) including the both side edge 30E in the width direction W.

That is, the end portions E1 and E2 both located inside the stretch region A in the width direction W are not provided colinearly in the width direction W.

To confirm whether the end portions E1, E2 inside the stretch region of the elastic members FG in the first region R1 in the width direction W may be disposed shifted from each other in the longitudinal direction L between the one outer side (for example, the left side of the both side edge 30E) including the both side edge 30E in the width direction W and the other outer side (for example, the right side of the both side edge 30E) including the both side edge 30E in the width direction W, a distance between the waist aperture H2 and each of the end portions E1, E2 disposed inside the elastic members FG in the first region R1 in the width direction W is measured. That is, the end portions E1 and E2 located inside the stretch region A in the width direction W can be regarded as being shifted from each other if the distance between the waist aperture H2 and the elastic members FG existing in the end portion E1 differs from the distance between the waist aperture H2 and the elastic members FG existing in the end portion E2.

This structure can decrease formation of wrinkles in the region where the absorber 30 is disposed in the width direction W, while preventing formation of a gap between the wearer and the absorber 30.

In the pants-type diaper 1 according to the present embodiment, the weight of an adhesive stacked on the region where the absorber 30 is disposed may be set lower than the weight of an adhesive stacked on the region where the elastic members FG are provided outside the region where the absorber 30 is disposed.

In this structure, the weight (basis weight) of the adhesive in the region where the absorber 30 that has a limited stretchability is disposed is lower than the weight (basis weight) of the adhesive stacked on the region other than the former region.

Therefore, the region where the absorber 30 is disposed can be kept flexible, while the front and rear waistline members 4 and 5 can be entirely flexible.

In the pants-type diaper 1 according to the present embodiment, the predetermined intervals between the elastic members FG disposed in the longitudinal direction L in the first region R may be in the range of 5 mm to 50 mm. If the predetermined interval is less than 5 mm, the wrinkle decreasing effect due to shifting in the longitudinal direction L of the elastic members FG decreases so that the wrinkles are likely to be formed in the width direction W. In addition, the extension stress increases, causing larger force to be needed to pull up the pants-type diaper 1. In contrast, if the predetermined interval is larger than 50 mm, fitting to the body of the wearer decreases.

In the pants-type diaper 1 according to the present embodiment, the absorber 30 may include a first layer absorber 30A disposed on the skin-abutting surface side and a second layer absorber 30B disposed closer to the non-skin-abutting surface side of the first layer absorber 30A is. The elastic members FG may be disposed outside the both side edges 30E in the width direction W in the region where at least one of the first layer and the second layer absorber 30A or 30B is disposed in the region where the absorber 30 is disposed.

In this structure, the elastic members FG are disposed outside the both side edges 30E in the width direction W in the region where at least one of the first layer and the second layer absorber 30A and 30B is disposed in the region where the absorber 30 is disposed. This prevents formation of wrinkles in the region where the absorber 30, which is formed by only one layer, is disposed, so that the wrinkles are easily formed. In addition, the effective absorbing width of the absorber 30 can be effectively provided.

Meanwhile, in the pants-type diaper 1 of the present embodiment, the absorber body 3 may have both side units S1 disposed outside the both side edges 30E in the width direction W of the absorber 30. As illustrated in FIG. 2, on both side units of the absorber body 3 including the both side units S1 in the width direction W, a leakage-preventing cuff LSG is provided including a fixed end LSG1 extending in the longitudinal direction L and a free end LSG2 in which a cuff elastic member is disposed in the longitudinal direction L to rise toward the skin-abutting surface side.

The leakage-preventing cuff LSG is formed of a liquid-impermeable side sheet and a leakage preventing elastic member. An end portion of the side sheet in the width direction W is folded so that the side sheet is doubled. The leakage-preventing elastic members are provided in an extended state in the longitudinal direction L at portions where the side sheet is doubled.

As illustrated in FIG. 2, the elastic members FG may be disposed outside the free end LSG2 in the width direction W in a portion where the front waistline member 4 (or the rear waistline member 5) overlaps the region where the free end LSG2 is disposed.

This structure can widen the support point of the leakage-preventing cuff LSG provided on the both side edges 30E of the absorber body 3 and improve rising characteristic of the leakage-preventing cuff LSG.

In the pants-type diaper 1 according to the present embodiment, as illustrated in FIG. 2, in the front waistline member 4, intervals of a plurality of leg-opening elastic members LG may be narrower than intervals of the plurality of elastic members FG in the longitudinal direction L. In addition, the leg-opening elastic members LG on the other outer side (for example, on the right side of the both side edges 30E) including the both side edge 30E in the width direction W may be disposed at positions identical with the position of the leg-opening elastic members LG, in the longitudinal direction L, on one outer side (for example, the left side of the both side edges 30E) including the both side edges 30E in the width direction W.

In this structure, when the wearer pulls up the waist aperture H2 by hand, the pulling-up force can easily be transmitted to the absorber 30 at the leg-aperture HI, which is located farthest from the hands and requires force to pull up the absorber 30, by decreasing the intervals of the leg-aperture elastic members LG. Further, the end portions E1 and E2 inside the stretch region in the width direction W are located at the same level of positions in the longitudinal direction L. Thus, the extension stress from the elastic members LG can be set evenly in the longitudinal direction L in the leg-apertures H1 after wearing. Fitting can therefore be attained without uncomfortable feeling, such as the feeling of biting into the buttocks.

### (Modification Example)

A modification example of the present invention is described below by focusing on what differs from the pants-type diaper 1 according to the first embodiment.

The pants-type diaper 1 according to the modification example may include the elastic members WG, FG, and LG made of sheet-like elastic members, instead of thread-like elastic members. The sheet-like elastic members are made of stretchable sheets formed by stretching and fixing one or more stretchable nonwoven fabrics or stretchable films, for example, between outer backsheet and the outer topsheet.

Alternatively, in the pants-type diaper 1 according to the modification example, the thread-like elastic members may be disposed such that the extension stress is provided evenly in the first and second regions R1 and R2, and another elastic member may be fixed in an extended state in the second region R2.

Further, the pants-type diaper 1 according to the modification example is provided such that the extension stress of the region where the absorber 30 is disposed is smaller than the extension stress of the first region R1. The extension stress of the first region R1 is smaller than the extension stress of the second region R2.

Such a difference in the extension stress may be provided, for example, by the difference in the type or the extension magnification of the thread-like elastic members that form the elastic members FG. Alternatively, the different extension stress may be achieved by a difference in the length of areas wherein the elastic members FG are respectively extended and fixed, by a different interval (pitch) of the thread-like elastic members that form the elastic members FG in the longitudinal direction L, or by different stress of the stretchable sheet.

### [INDUSTRIAL APPLICABILITY]

As described above, the present invention can provide the pants-type diaper that can achieve a wider effective absorbing range of the absorber, adjust the stretchability of the elastic members in the width direction, and improve the wearing feeling of the absorber.

### [REFERENCE SIGNS LIST]

1: Pants-type diaper
10, 41: Backsheet
20, 40: Topsheet
3: Absorber body
30: Absorber
30A: First absorber
30B: Second absorber
30E: Both side edges
4: Front waistline member
5: Rear waistline member
50: Bonded portion
FG, WG, LG: Elastic member
S1: Both side units
LSG: Leakage-preventing cuff
LSG1: Fixed end
LSG2: Free end
H1: Leg aperture
H2: Waist aperture
R1: First region
R2: Second region

## Claims

1. A pants-type diaper (1), comprising:
a width direction (W) and a longitudinal direction (L) which are orthogonal to each other;
a front waistline member (4);
a rear waistline member (5) ;
an absorber body (3) extending from the front waistline member to the rear waistline member;
an absorber (30) disposed in the absorber body; and
a plurality of elastic members (FG,WG) disposed at predetermined intervals in the longitudinal direction in the front waistline member (4) and the rear waistline member (5), wherein
at least one of the front waistline member (4) and the rear waistline member (5) includes a first region (R1) including both side edges along the longitudinal direction of the region where the absorber (30) is disposed and outside in the width direction, and a second region (R2) provided outside the first region in the width direction, and
extension stress of the first region (R1) is smaller than extension stress of the second region (R2), wherein
inner edge portions (E1, E2) of the elastic members (FG) provided in the first region (R1) are disposed shifted from each other in the longitudinal direction between the one outer side including the both side edge in the width direction and the other outer side including the both side edge in the width direction.

2. The pants-type diaper according to claim 1, wherein
the elastic members (FG, WG) and an adhesive are stacked between a pair of sheet members in at least one of the front waistline member (4) and the rear waistline member (5), and
the basis weight of the adhesive in the region where the absorber (30) is disposed is smaller than a basis weight of the adhesive in a region where the elastic members (FG, WG) are provided and is outside the region where the absorber (30) is disposed.

3. The pants-type diaper according to any one of claims 1 to 2, wherein
the predetermined intervals between the elastic member FG disposed in the longitudinal direction L in the first region (R1) are in a range of 5 mm to 50 mm.

4. The pants-type diaper according to any one of claims 1 to 3, wherein
the absorber (30) includes a first-layer absorber (30A) disposed on a skin-abutting surface side and a second-layer absorber (30B) disposed closer to a non-skin-abutting surface side than the first layer absorber is, and
the elastic members (FG, WG) are disposed outside in the width direction of a region including the both side edges where at least one of the first-layer absorber and the second-layer absorber is disposed in the region where the absorber is disposed.

5. The pants-type diaper according to any one of claims 1 to 4, wherein
the absorber body includes both side units (S1) located outside the both side edges (30E) of the absorber (30) in the width direction,
a leakage-preventing cuff (LSG) having a fixed end (LSG1) extending in the longitudinal direction and a free end (LSG2) at which a cuff elastic member is disposed in the longitudinal direction to rise toward a skin-abutting surface side, on both side units of the absorber body including the both side units, and
the elastic members (FG, WG) are disposed outside the free end in the width direction in a region where the front waistline member or the rear waistline member overlaps the region where the free end is located.

6. The pants-type diaper according to any one of claims 1 to 5, wherein intervals of a plurality of leg-opening elastic members (LG) disposed on a leg-aperture side are narrower than intervals of the elastic members in the longitudinal direction, and
the leg-opening elastic members on the other outer side including the both side edge in the width direction are disposed at positions identical with the position of the leg-opening elastic members, in the longitudinal direction, on the one outer side including the both side edges in the width direction.

## Patentansprüche

1. Hosenartige Windel (1), umfassend:
eine Breitenrichtung (W) und eine Längsrichtung (L), die orthogonal zueinander sind;
ein vorderes Taillenelement (4);
ein hinteres Taillenelement (5);
einen Absorberkörper (3), der sich von dem vorderen Taillenelement zu dem hinteren Taillenelement erstreckt;
einen Absorber (30), der in dem Absorberkörper angeordnet ist; und
eine Vielzahl von elastischen Elementen (FG, WG), die in vorbestimmten Abständen in Längsrichtung in dem vorderen Taillenelement (4) und dem hinteren Taillenelement (5) angeordnet sind, wobei
mindestens eines des vorderen Taillenelements (4) und des hinteren Taillenelements (5) einen ersten Bereich (R1) mit beiden Seitenkanten entlang der Längsrichtung des Bereichs, in dem der Absorber (30) angeordnet ist, und außerhalb in der Breitenrichtung, und einen zweiten Bereich (R2), der außerhalb des ersten Bereichs in der Breitenrichtung angeordnet ist, umfasst, und die Dehnungsspannung des ersten Bereichs (R1) kleiner ist als die Dehnungsspannung des zweiten Bereichs (R2), wobei
Innenkantenteile (E1, E2) der im ersten Bereich (R1) vorgesehenen elastischen Elemente (FG) in Längsrichtung zwischen der einen Außenseite, die die beiden Seitenkanten in Breitenrichtung umfasst, und der anderen Außenseite, die die beiden Seitenkanten in Breitenrichtung umfasst, gegeneinander versetzt angeordnet sind.

2. Hosenartige Windel gemäß Anspruch 1, wobei die Hose
die elastischen Elemente (FG, WG) und ein Klebstoff zwischen einem Paar Lagenelementen in mindestens einem der vorderen Taillenelemente (4) und hinteren Taillenelemente (5) gestapelt sind, und das Flächengewicht des Klebstoffs in dem Bereich, in dem der Absorber (30) angeordnet ist, kleiner ist als ein Flächengewicht des Klebstoffs in einem Bereich, in dem die elastischen Elemente (FG, WG) vorgesehen sind, und außerhalb des Bereichs liegt, in dem der Absorber (30) angeordnet ist.

3. Hosenartige Windel gemäß einem der Ansprüche 1 bis 2, wobei
die vorbestimmten Abstände zwischen dem in Längsrichtung (L) im ersten Bereich (R1) angeordneten elastischen Element (FG) in einem Bereich von 5 mm bis 50 mm liegen.

4. Hosenartige Windel gemäß einem der Ansprüche 1 bis 3, wobei
der Absorber (30) einen Erstschichtabsorber (30A), der auf einer hautanliegenden Oberflächenseite angeordnet ist, und einen Zweitschichtabsorber (30B), der näher an einer nicht hautanliegenden Oberflächenseite angeordnet ist, als der Erstschichtabsorber, umfasst, und
die elastischen Elemente (FG, WG) außerhalb in Breitenrichtung eines Bereichs mit den beiden Seitenkanten angeordnet sind, in dem mindestens einer der Erstschichtabsorber und der Zweitschichtabsorber in dem Bereich angeordnet ist, in dem der Absorber angeordnet ist.

5. Hosenartige Windel gemäß einem der Ansprüche 1 bis 4, wobei
der Absorberkörper beide Seiteneinheiten (S1) umfasst, die außerhalb der beiden Seitenkanten (30E) des Absorbers (30) in Breitenrichtung angeordnet sind,
eine leckageverhindernde Manschette (LSG) mit einem festen Ende (LSG1), das sich in Längsrichtung erstreckt, und einem freien Ende (LSG2), an dem ein elastisches Manschettenelement in Längsrichtung angeordnet ist, um in Richtung einer hautanliegenden Oberflächenseite zu steigen, auf beiden Seiteneinheiten des Absorberkörpers einschließlich der beiden Seiteneinheiten, und
die elastischen Elemente (FG, WG) außerhalb des freien Endes in Breitenrichtung in einem Bereich angeordnet sind, in dem das vordere Taillenelement oder das hintere Taillenelement den Bereich überlappt, in dem sich das freie Ende befindet.

6. Hosenartige Windel gemäß einem der Ansprüche 1 bis 5, wobei
die Abstände einer Vielzahl von Beinöffnungs elastischen Elementen (LG), die auf einer Beinöffnungsseite angeordnet sind, schmaler sind als die Abstände der elastischen Elemente in Längsrichtung, und
die Beinöffnungs elastischen Elemente auf der anderen Außenseite, die die beiden Seitenkanten in Breitenrichtung umfassen, an Positionen angeordnet sind, die identisch mit der Position der Beinöffnungselastischen Elemente in Längsrichtung sind, auf der einen Außenseite, die die beiden Seitenkanten in Breitenrichtung umfasst.

## Revendications

1. Couche de type culotte (1), comprenant :
une direction de largeur (W) et une direction longitudinale (L) qui sont orthogonales l'une par rapport à l'autre ;
un élément de taille avant (4) ;
un élément de taille arrière (5) ;
un corps absorbant (3) s'étendant de l'élément de taille avant à l'élément de taille arrière ;
un élément absorbant (30) disposé dans le corps absorbant ; et
une pluralité d'éléments élastiques (FG, WG) disposés à des intervalles prédéterminés dans la direction longitudinale dans l'élément de taille avant (4) et l'élément de taille arrière (5), dans laquelle
au moins l'un de l'élément de taille avant (4) et de l'élément de taille arrière (5) inclut une première région (R1) incluant les deux bords latéraux le long de la direction longitudinale de la région où l'élément absorbant (30) est disposé et se trouve à l'extérieur dans la direction de largeur, et une seconde région (R2) prévue à l'extérieur de la première région dans la direction de largeur, et
la contrainte d'extension de la première région (R1) est inférieure à la contrainte d'extension de la seconde région (R2), dans laquelle
des parties de bord intérieures (E1, E2) des éléments élastiques (FG) prévus dans la première région (R1) sont disposées de manière décalée les unes par rapport aux autres dans la direction longitudinale entre l'un côté extérieur incluant les deux bords latéraux dans la direction de largeur et l'autre côté extérieur incluant les deux bords latéraux dans la direction de largeur.

2. Couche de type culotte selon la revendication 1, dans laquelle
les éléments élastiques (FG, WG) et un adhésif sont empilés entre une paire d'éléments en feuille dans au moins l'un de l'élément de taille avant (4) et de l'élément de taille arrière (5), et
le poids de base de l'adhésif dans la région où l'élément absorbant (30) est disposé est inférieur à un poids de base de l'adhésif dans une région où les éléments élastiques (FG, WG) sont prévus et se trouve à l'extérieur de la région où l'élément absorbant (30) est disposé.

3. Couche de type culotte selon l'une quelconque des revendications 1 à 2, dans laquelle
les intervalles prédéterminés entre l'élément élastique FG disposé dans la direction longitudinale L dans la première région (R1) sont compris entre 5 mm et 50 mm.

4. Couche de type culotte selon l'une quelconque des revendications 1 à 3, dans laquelle l'élément absorbant (30) inclut un élément absorbant de première couche (30A) disposé sur un côté de surface en contact avec la peau et un élément absorbant de seconde couche (30B) disposé plus près d'un côté de surface n'étant pas en contact avec la peau que l'élément absorbant de première couche, et les éléments élastiques (FG, WG) sont disposés à l'extérieur dans la direction de largeur d'une région incluant les deux bords latéraux où au moins l'un de l'élément absorbant de première couche et de l'élément absorbant de seconde couche est disposé dans la région où l'élément absorbant est disposé.

5. Couche de type culotte selon l'une quelconque des revendications 1 à 4, dans laquelle
le corps absorbant inclut les deux unités latérales (S1) situées à l'extérieur des deux bords latéraux (30E) de l'élément absorbant (30) dans la direction de largeur,
un manchon antifuite (LSG) ayant une extrémité fixe (LSG1) s'étendant dans la direction longitudinale et une extrémité libre (LSG2) au niveau de laquelle un élément élastique de manchon est disposé dans la direction longitudinale pour s'élever vers un côté de la surface en contact avec la peau, sur les deux unités latérales du corps absorbant incluant les deux unités latérales, et
les éléments élastiques (FG, WG) sont disposés à l'extérieur de l'extrémité libre dans la direction de largeur dans une région où l'élément de taille avant ou l'élément de taille arrière recouvre la région où se trouve l'extrémité libre.

6. Couche de type culotte selon l'une quelconque des revendications 1 à 5, dans laquelle
des intervalles d'une pluralité d'éléments élastiques d'ouverture de jambe (LG) disposés sur un côté d'ouverture de jambe sont plus étroits que les intervalles des éléments élastiques dans la direction longitudinale, et
les éléments élastiques d'ouverture de jambe sur l'autre côté extérieur incluant les deux bords latéraux dans la direction de largeur sont disposés à des positions identiques à la position des éléments élastiques d'ouverture de jambe, dans la direction longitudinale, sur l'un côté extérieur incluant les deux bords latéraux dans la direction de largeur.
